(19) Europäisches Patentamt
European Patent Office
Office européen des brevets

(11) EP 0 672 664 B1

(12) **FASCICULE DE BREVET EUROPEEN**

(45) Date de publication et mention de la délivrance du brevet:
**21.03.2001 Bulletin 2001/12**

(51) Int Cl.$^7$: **C07D 311/08**

(21) Numéro de dépôt: **95400517.9**

(22) Date de dépôt: **10.03.1995**

(54) **Spherules de coumarine et/ou dérivés et leur procédé d'obtention**

Kleine Kugeln von Kumarin und/oder Derivate und Verfahren zu deren Herstellung

Little spheres of coumarin and/or derivatives and process for the preparation thereof

(84) Etats contractants désignés:
**AT BE CH DE DK ES FR GB IE IT LI NL PT SE**

(30) Priorité: **16.03.1994 FR 9403094**

(43) Date de publication de la demande:
**20.09.1995 Bulletin 1995/38**

(73) Titulaire: **RHODIA CHIMIE**
**92408 Courbevoie Cédex (FR)**

(72) Inventeurs:
- **Cervos, Eric**
**F-69009 Lyon (FR)**
- **Labourt-Ibarre, Pierre**
**F-69006 Lyon (FR)**
- **Statiotis, Eraclis**
**F-38280 Villette d'Anthon (FR)**

(74) Mandataire: **Dutruc-Rosset, Marie-Claude et al**
**Rhodia Services,**
**Direction de la Propriété Industrielle,**
**40, rue de la Haie-Coq**
**93306 Aubervilliers Cedex (FR)**

(56) Documents cités:
**WO-A-89/10566**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

EP 0 672 664 B1

## Description

La présente invention a pour objet une nouvelle présentation de la coumarine et de ses dérivés. Plus précisément, l'invention a pour objet des sphérules de coumarine et/ou dérivés. L'invention se rapporte aussi à la préparation desdites sphérules.

La coumarine et ses dérivés sont des produits largement utilisés dans le domaine de la parfumerie. De plus, la coumarine trouve des applications dans d'autres domaines tels que par exemple, la pharmacie. Il s'en suit que c'est un produit de grande consommation.

La coumarine est actuellement disponible sur le marché sous la forme d'une poudre cristallisée présentant des cristaux de type plaquette ou pavé. Les inconvénients qui en résultent sont ceux liés à la manipulation d'une poudre.

Un premier objectif de l'invention est de fournir une nouvelle forme ou présentation de la coumarine qui ne génère pas de poussières et qui ne motte pas au cours du stockage.

Un autre objectif est qu'elle présente également de bonnes propriétés d'écoulement.

Un objectif également est qu'elle possède une vitesse de dissolution améliorée par rapport à celle requise lors d'une application ultérieure.

Précisément, la présente invention propose une nouvelle forme ou présentation de la coumarine et de ses dérivés, répondant à ces exigences.

La présente invention a pour objet des sphérules de la coumarine et/ou dérivés.

Dans l'exposé de la présente invention, on entend par "sphérules", des particules solides à forte sphéricité.

La caractéristique du procédé de l'invention, en vue de préparer des sphérules de coumarine et/ou dérivés est de faire fondre si nécessaire la coumarine et/ou ses dérivés, puis de fragmenter la masse fondue en gouttelettes, et solidifier les gouttelettes obtenues dans un courant gazeux de refroidissement de telle sorte qu'elles se solidifient en sphérules qui sont ensuite récupérées.

Une variante préférée du procédé de l'invention consiste à faire fondre si nécessaire la coumarine et/ou ses dérivés, puis à faire passer la masse fondue dans une buse de façon à former des gouttelettes, à solidifier ces dernières en les laissant tomber dans une tour à contre-courant d'un gaz froid, puis à récupérer les sphérules obtenues.

Le procédé de l'invention est parfaitement bien adapté pour la préparation de sphérules de coumarine mais il convient également pour tout dérivé de la coumarine dans la mesure où il présente un point de fusion compris entre 50°C et 200°C, de préférence compris entre 50°C et 100°C.

Comme exemples de composés, coumarine et dérivés auxquels peut s'appliquer le procédé de l'invention, on peut mentionner ceux répondant à la formule (I) suivante :

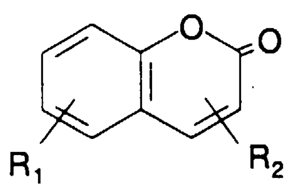

(I)

dans ladite formule (I), $R_1$, $R_2$, identiques ou différents représentent :

- un atome d'hydrogène,
- un radical alkyle linéaire ou ramifié ayant de 1 à 4 atomes de carbone,
- un radical alkoxy linéaire ou ramifié ayant de 1 à 4 atomes de carbone.

Les composés de formule (I) mis en oeuvre préférentiellement sous forme de sphérules sont ceux répondant à la formule (I) dans laquelle $R_1$, $R_2$, identiques ou différents représentent un atome d'hydrogène, un radical méthyle ou méthoxy.

Comme exemples spécifiques de composés de formule (I), on peut citer tout particulièrement :

- la coumarine,
- la 3-méthylcoumarine,
- la 4-méthylcoumarine,
- la 5-méthylcoumarine,
- la 6-méthylcoumarine,
- la 7-méthylcoumarine,
- la 8-méthylcoumarine,

- la 4-méthoxycoumarine,
- la 4-méthyl-7-méthoxycoumarine,
- la 4-méthyl-7-éthoxycoumarine.

Dans le présent texte, on utilise ci-après, le terme "coumarine" de manière générique et l'on désigne par ce terme aussi bien la coumarine que ses dérivés répondant à la formule (I).

Les sphérules obtenues selon l'invention présentent des caractéristiques physico-chimiques qui leur sont propres.

Les définitions et les méthodes de détermination des caractéristiques données ci-après, sont précisées dans les exemples.

La détermination des tailles se fait par passage sur des tamis métalliques.

Les sphérules de coumarine sont sous la forme de billes de couleur blanche. Elles présentent une taille de particules essentiellement sous forme sphériques, ayant un diamètre qui peut être choisi, grâce au procédé de l'invention, dans une large gamme. Ainsi, la taille des particules peut s'échelonner entre 100 μm et 2000 μm mais se situe, de préférence, entre 300 μm et 1000 μm.

Généralement, la taille des particules exprimée par le diamètre moyen ($d_{50}$) varie de 300 μm à 2 000 μm, de préférence entre 500 μm et 1 500 μm et encore plus préférentiellement entre 700 μm et 1 200 μm. On définit le diamètre moyen comme étant tel que 50 % en poids des particules ont un diamètre supérieur ou inférieur au diamètre moyen.

Les figures 1 à 5 représentent des photographies prises au microscope électronique à balayage qui montrent la morphologie du type sphérule de la coumarine obtenue selon l'invention.

Sur le produit obtenu, on observe une répartition granulométrique uniforme.

Les sphérules ont une densité qui peut être choisie plus ou moins élevée, en adaptant les paramètres de procédé. On peut ainsi moduler la qualité des produits suivant la demande du marché. Ainsi, il est possible que la densité apparente (non tassée) des sphérules varie 0,25 et 0,8, de préférence, entre 0,4 et 0,7.

Les sphérules de coumarine de l'invention présentent une résistance à la compression comprise entre 500 et 10 000 $N/m^2$ , de préférence comprise entre 1 000 et 5 000 $N/m^2$, sous une contrainte inférieure à 10 000 $N/m^2$.

Elles présentent une cohésion adaptée à de bonnes propriétés d'écoulement.

L'invention réside donc en des sphérules de coumarine qui bien qu'ayant une forme physique leur permettant de résister à l'attrition, conservent une vitesse de dissolution rapide, lors de leur utilisation. Leur vitesse de dissolution dans l'éthanol qui constitue un test utilisé en galénique, est inférieure à la vitesse de dissolution de la poudre cristallisée correspondante. On précisera, que dans le cas de la coumarine proprement dite, la vitesse de dissolution dans l'éthanol, des sphérules de coumarine est inférieure à 170 secondes, de préférence, comprise entre 100 et 170 secondes, et encore plus préférentiellement comprise entre 100 et 130 secondes.

La structure originale des produits de l'invention est obtenue grâce à un procédé de fabrication parfaitement adapté.

Le procédé de l'invention de préparation de sphérules de coumarine consiste à faire fondre si nécessaire la coumarine et/ou ses dérivés, puis à fragmenter la masse fondue en gouttelettes, et à solidifier les gouttelettes obtenues dans un courant gazeux de refroidissement de telle sorte que les gouttelettes obtenues se solidifient en sphérules qui sont ensuite récupérées.

Dans le procédé de l'invention, on met en oeuvre de la coumarine fondue.

On peut envisager d'alimenter directement la coumarine fondue provenant d'une ligne de fabrication.

Il est également possible de prévoir une étape de procédé de l'invention qui consiste à faire fondre la coumarine. A cet effet, on chauffe le produit à sa température de fusion. De préférence, on porte le produit à une température légèrement supérieure à sa température de fusion, de préférence supérieure à au plus 5°C par rapport à son point de fusion. Pour la coumarine spécifiquement, la température à laquelle est portée celle-ci, est choisie entre 70°C et 75°C.

Cette opération est effectuée, généralement sous agitation.

Dans une étape suivante, on transforme la masse fondue, en gouttelettes. Cette opération peut être réalisée au moyen de tout dispositif de fragmentation, par exemple, par une buse plate à orifice(s) circulaire(s).

Un mode de réalisation préférentielle de l'invention consiste à former les gouttelettes par passage de la masse fondue au travers d'un orifice et tout particulièrement par passage au travers d'une buse.

L'opération suivante est d'assurer le "figeage" des gouttelettes en sphérules par contact avec un gaz froid dont la température est choisie entre -30°C et 20°C, de préférence, entre -20°C et 10°C.

Le gaz froid est de préférence l'air mais l'invention n'exclut pas un quelconque gaz dans la mesure où il est inerte vis-à-vis de la coumarine. On peut choisir l'azote mais d'une manière générale, on préfère l'air et, plus préférentiellement, de l'air appauvri en oxygène (par exemple à 10 %).

D'une manière préférentielle, on envoie le courant gazeux froid, à contre-courant du flux de matière.

Le temps de séjour qui est la durée entre la formation de la gouttelette à la sortie de la buse et son arrivée

dans le système de récupération se situe avantageusement, entre 1 et 10 secondes et plus préférentiellement entre 1 et 3 secondes.

Une façon d'obtenir le temps de séjour désiré, est de laisser tomber les gouttelettes dans une tour à contre-courant d'un gaz froid tel que précité.

En fin de réaction, on récupère les sphérules par tout moyen connu, par exemple, par gravité dans un bac de récupération ou, de préférence, selon la technique de lit fluide.

Les sphérules de coumarine ainsi obtenues selon le procédé de l'invention présentent les caractéristiques explicitées ci-dessus.

Un avantage très intéressant du procédé de l'invention est non seulement une nouvelle présentation de la coumarine mais également l'obtention d'une purification du produit mis en oeuvre. En effet, on a constaté une baisse du taux d'impuretés dans les sphérules de coumarine obtenues par rapport à la poudre de départ. Les courbes d'analyse thermique différentielle (déterminées sur appareil Perkin-Eimer®) qui constituent la figure 6, le mettent nettement en évidence.

La figure 6 représente un graphe sur lequel sont portées deux courbes de variation du flux thermique (exprimé en w/g) en fonction de la température de fusion (exprimée en °C) : la courbe (A) correspond à la courbe obtenue en mettant en oeuvre une coumarine du commerce sous forme de poudre et la courbe (B) est obtenue avec la coumarine préparée avec la même poudre de départ mais mise en forme selon l'invention, sous forme de sphérules.

La différence de hauteur des pics et la raideur du front de la courbe (B) par rapport à la courbe (A) indique une meilleure pureté de la coumarine présentée selon l'invention.

Par ailleurs, on a constaté que lorsque le gaz de refroidissement introduit de préférence, à contre-courant de la matière présente une température supérieure à 10°C, on obtient des sphérules présentant une morphologie dendritique conduisant à une baisse de densité apparente, généralement inférieure à 0,35, et située de préférence, entre 0,25 et 0,35. Dans ce cas, la vitesse de dissolution des sphérules est améliorée.

En ce qui concerne l'appareillage utilisé pour mettre en oeuvre le procédé de l'invention, il est composé de deux ensembles : un premier ensemble de mise en forme des sphérules et un deuxième ensemble de récupération des sphérules.

Le premier ensemble comprend un bac de stockage de préférence agité lorsque la coumarine provient d'une ligne de fabrication ou bien un fondoir permettant de fondre la coumarine et une enceinte qui est généralement, une tour d'une hauteur allant de préférence entre 4 et 8 mètres, comprenant dans sa partie supérieure, un dispositif de fragmentation en gouttelettes, de préférence une buse et équipée dans sa partie inférieure d'une ou plusieurs arrivées d'un courant gazeux froid transformant ainsi le bas de la tour en une tour de refroidissement.

La coumarine est introduite par une trémie à double vis, dans un fondoir qui est un réacteur équipé d'un système permettant de réguler la température, par exemple, une double enveloppe, afin de maintenir la coumarine à l'état fondu.

La buse utilisée peut être une buse à un seul trou ou une buse multi-trous avec un nombre de trous qui peut varier entre 1 et 100 trous.

On peut utiliser un système comprenant plusieurs buses, par exemple, 2 buses de préférence amovibles, en parallèle.

Le diamètre des perforations de la buse est fonction de la taille des sphérules désirées. Il peut être de 100 à 1 000 µm mais il est choisi, de préférence, entre 200 et 600 µm.

La taille de la perforation est toujours inférieure à la taille de la sphérule obtenue. Ainsi, on utilise une buse présentant des perforations d'environ 200 µm pour obtenir des sphérules présentant un diamètre moyen de 600 µm.

La buse utilisée peut être une buse statique mais il est possible de faire appel à une buse soumise à un système de vibration électrique de haute fréquence, par exemple, de 500 à 10000 hertz.

Le produit fondu arrive dans la buse soit à l'aide d'une pompe volumétrique ou soit par une surpression qui est assurée par un courant gazeux, de préférence, un courant d'azote. La surpression par rapport à la pression atmosphérique est de 5 à 500 %.

La buse est maintenue à une température comprise entre 70 et 80°C.

Au niveau de la buse, il est possible mais non indispensable d'établir un courant gazeux, de préférence un co-courant d'air avec le jet sortant de la buse. Ce courant d'air a, de préférence, une température comprise entre la température ambiante et 60°C. Le présence de ce co-courant gazeux permet d'obtenir une meilleure régularité de la dimension des sphérules et évite la coalescence des gouttes.

Dans la partie médiane de la tour, il peut y avoir sur le paroi interne de la tour, présence de chicanes et de grilles permettant l'homogénéisation du flux gazeux.

Dans le bas de la tour, on introduit un courant de gaz froid, de préférence un courant d'air froid pour assurer le "figeage" des gouttelettes en sphérules. Ce courant d'air a de préférence une température comprise entre -30°C et 20°C, de préférence, comprise entre -20°C et 10°C.

L'air froid sort, de préférence, de la tour, en dessous de la buse, à une distance représentant un dixième de

la hauteur totale de la zone de refroidissement.

Le système de récupération des sphérules, en bas de la tour, peut être constitué d'un bac de récupération mais il est préférable de faire appel à un dispositif permettant d'assurer la fluidisation du lit de particules. Il est constitué par un bac, de préférence cylindrique, comportant dans sa partie inférieure, une grille au travers de laquelle est envoyé un courant gazeux, de préférence de l'air appauvri en oxygène. Le débit d'air, qui dépend de la taille des particules, doit être tel qu'il maintient les particules en suspension. On précise, à titre d'exemple, qu'il est de 5 à 30 $m^3/h$ pour un diamètre de tour de 80 mm.

Dans cette partie de l'appareil, on poursuit le refroidissement car la température de l'air envoyé est à la même température (± 5°C) que la température du courant d'air froid envoyé, en bas de tour.

Le dispositif de fluidisation dispose d'une sortie permettant l'évacuation des sphérules.

Un mode de réalisation pratique de l'invention est illustré par le dessin annexé sous forme de figure 7.

La figure 7 est une vue latérale schématique d'un appareil adapté à la mise en oeuvre de l'invention.

L'appareil utilisé est constitué de deux parties : la partie supérieure ou tour de prilling (A) et la partie inférieure qui schématise un dispositif de fluidisation (B).

La poudre de coumarine est introduite dans le pot (2) : le deuxième pot (1) contient une solution de lavage (par exemple du méthanol) du système d'alimentation, en fin d'opération.

L'azote (5) est admis en surpression dans les bacs à réserve (1) ou (2).

La tour d'une hauteur de 4 mètres, comprend une buse (4) dans sa partie supérieure et est équipée dans sa partie inférieure d'une arrivée d'un courant d'air froid (3).

L'air de refroidissement introduit en (3) ressort au point (6), en dessous de la sortie de la buse (4).

Dans la partie médiane de la tour, des chicanes sont introduites en (8) ainsi qu'une grille de forme annulaire, d'homogénéisation du flux d'air en (9).

Dans la partie inférieure (8) de la tour, de préférence de forme évasée assurant ainsi une meilleure collecte des sphérules, est adapté un dispositif de fluidisation comprenant une arrivée d'air froid en (9) et une sortie (10) permettant l'évacuation des sphérules obtenues.

On donne ci-après, des exemples de réalisation pratique de l'invention.

Avant de détailler les exemples, on précise les méthodes utilisées pour la détermination des différentes caractéristiques des produits obtenus.

- <u>la densité apparente non tassée :</u>

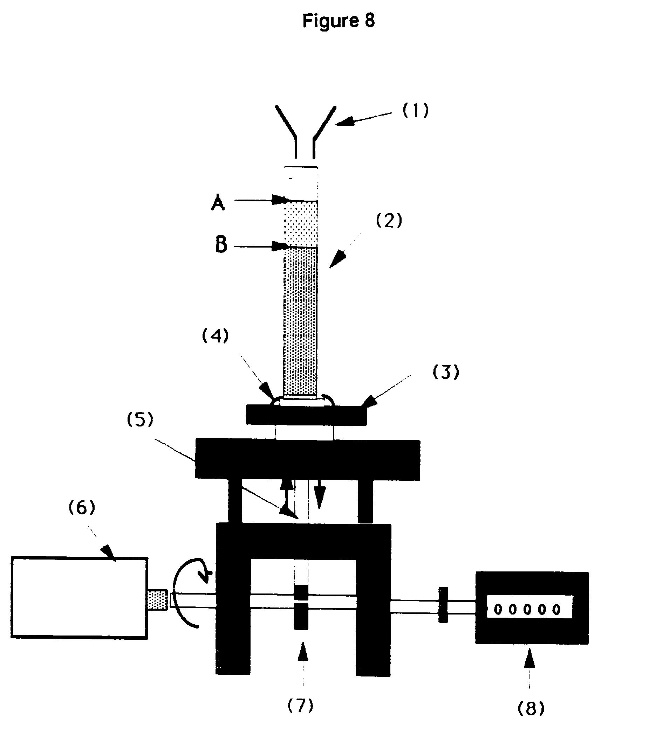

On la mesure sur un appareil illustré par la figure 8.

On commence par peser l'éprouvette vide (2).

On introduit dans l'éprouvette (2) la poudre à mesurer à l'aide de l'entonnoir (1), de manière à ce que le haut du lit de poudre vienne au ras du haut de l'éprouvette jaugée à 250 $cm^3$ (niveau A).

On détermine la masse de la poudre par pesée de l'éprouvette pleine.

On assujettit l'éprouvette sur le support (3) par l'intermédiaire de pinces (4).

On met à zéro le compteur (8) qui totalise le nombre de coups imposés au fond de l'éprouvette.

L'éprouvette est soumise à des chocs verticaux appliqués à sa base par l'intermédiaire d'un marteau (5) actionné par un moteur (6) via une came (7). On arrête l'opération lorsque le volume obtenu est constant (niveau B).

On enregistre l'évolution du volume apparent lu sur les graduations de l'éprouvette en fonction du nombre de coups appliqués à l'aide d'un marteau.

On obtient une courbe expérimentale de tassement.

Volume apparent = f (nombre de coups) que l'on transforme en une courbe densité apparente = f (nombre de coups).

On détermine la densité apparente selon la relation :

$$\text{densité apparente} = \frac{\text{masse de la poudre introduite (g)}}{\text{volume apparent (cm}^3\text{)}}$$

- <u>la résistance à la compression :</u>

La résistance à la compression (fc) des produits de l'invention est mesurée en cellule de Jenike, selon la méthode explicitée par L. SVAROSVSKY dans Powder Testing Guide : Methods of measuring the physical properties of bulk powders - Elsevier Applied Science pp. 49-52 (1987).

- la vitesse de dissolution dans l'éthanol :

Dans une éprouvette, on dissout à 25°C sous agitation (50 tours/minute), x g de poudre (sphérules ou poudre cristallisée) dans l'éthanol à raison de 0,02 % en poids.
On suit l'évolution de l'absorbance de la solution d'un rayonnement UV (spectophotomètre) en fonction du temps. Le temps de dissolution exprimé en secondes est obtenu lorsque l'absorbance se stabilise à sa valeur finale.
Les exemples qui suivent, illustrent l'invention, sans pour autant la limiter.

EXEMPLES

On définit, ci-après, le protocole opératoire qui sera repris dans les exemples suivants.
On met en oeuvre 2000 g de coumarine cristallisée, sous forme poudre, ayant les caractéristiques suivantes :

- densité apparente non tassée = 0,683
- vitesse de dissolution dans l'éthanol = 174 s.

Le procédé de l'invention est mis en oeuvre dans l'appareillage décrit ci-dessus et schématisé par la figure 7.
La buse, soumise ou non a des vibrations, présente des caractéristiques précisées dans les exemples suivants.
On introduit la poudre de coumarine dans le pot (2), le pot (1) contenant une solution de lavage du système d'alimentation (méthanol).
On fond la coumarine dans le fondoir par chauffage à l'aide d'eau chaude circulant dans la double enveloppe. La température du produit est de 75°C en (2) et de 71°C en (4), en sortie de buse. Le débit du produit est précisé ci-après.
On introduit en (3) de l'air de refroidissement à un débit de 1000 $m^3$/h, soit une vitesse dans la tour de 0,7 m/s. L'air ressort en (6).
Les températures de l'air à l'entrée de la tour (3) et à la sortie de la tour en (6) sont précisées dans un tableau récapitulatif donné dans chaque exemple.
Il en est de même pour la température de l'air de fluidisation en (9).
Les sphérules obtenues sont collectées en (8) et évacuées en (10).

Exemple 1

1 - Les sphérules de coumarine sont préparées dans un appareillage tel que schématisé par la figure 7, comportant une buse à 7 trous, ayant un diamètre de trous de 250 µm. Le rapport L/D est de 1 ; L représentant la longueur de l'orifice et D le diamètre de l'orifice.

Le procédé mis en oeuvre est tel que décrit ci-dessus : les conditions de marche étant précisées dans le tableau I suivant :

Tableau I

| | |
|---|---|
| Fréquence de vibration de la buse (4) en hertz | 1160 |
| Surpression de l'azote (4) en bars | 0,202 |
| Débit du produit à la sortie de la buse (4) en kg/h | 5,60 |
| Température de l'air à l'entrée de la tour (3) en °C | - 10°C |
| Température de l'air à la sortie de la tour (6) en °C | - 6°C |
| Température de l'air du lit fluide (9) en °C | - 5 °C |

Après 12 minutes de fonctionnement, on récupère 840 g de sphérules ayant un diamètre moyen ($d_{50}$) de 800 µm.

2 - La figure 1 prise au microscope (G = 100) montre la morphologie de la coumarine obtenue sous la forme de billes.

Les autres caractéristiques physico-chimiques sont les suivantes :

- densité apparente non tassée = 0,47
- vitesse de dissolution dans l'éthanol = 125 s.

<u>Exemple 2</u>

1 - La préparation des sphérules de coumarine est effectuée comme dans l'exemple 1 : les modifications des paramètres de procédé étant précisées dans le tableau suivant:

Tableau II

| | |
|---|---|
| Fréquence de vibration de la buse (4) en hertz | 5600 |
| Surpression de l'azote (4) en bars | 0,222 |
| Débit du produit à la sortie de la buse (4) en kg/h | 5,60 |
| Température de l'air à l'entrée de la tour (3) en °C | 10 |
| Température de l'air à la sortie de la tour (6) en °C | 11 |
| Température de l'air du lit fluide (9) en °C | 10 |

Après 9 minutes de fonctionnement, on récupère 410 g de sphérules de coumarine ayant un diamètre moyen ($d_{50}$) de 700 µm.

2 - Le produit obtenu présente la morphologie illustrée par la figure 2 (G = 100) et les caractéristiques suivantes :

- densité apparente non tassée = 0,43
- vitesse de dissolution dans l'éthanol = 130 s.

<u>Exemple 3</u>

1 - Les sphérules de coumarine sont préparées dans un appareillage tel que schématisé par la figure 7, comportant une buse à 7 trous, ayant un diamètre de trous de 400 µm ; le rapport L/D étant de 3.

Le procédé mis en oeuvre est tel que décrit ci-dessus : les conditions de marche étant précisées dans le tableau III suivant :

Tableau III

| | |
|---|---|
| Fréquence de vibration de la buse (4) en hertz | 1540 |
| Surpression de l'azote (4) en bars | 0,08 |
| Débit du produit à la sortie de la buse (4) en kg/h | 7,00 |
| Température de l'air à l'entrée de la tour (3) en °C | 12 |
| Température de l'air à la sortie de la tour (6) en °C | 13 |
| Température de l'air du lit fluide (9) en °C | 10 |

Après 12 minutes de fonctionnement, on récupère 910 g de sphérules de coumarine ayant un diamètre moyen ($d_{50}$) de 1130 µm.

2 - Le produit obtenu présente la morphologie dendritique illustrée par la figure 3 (G = 60) et les caractéristiques suivantes :

- densité apparente non tassée = 0,27
- résistance à la compression (fc) = 1,6 $kN/m^2$ sous une pression de 4,8 $kN/m^2$
- vitesse de dissolution dans l'éthanol = 104 s.

Exemple 4

1 - La préparation des sphérules de coumarine est effectuée comme dans l'exemple 3 : les modifications des paramètres de procédé étant données dans le tableau IV suivant.

Tableau IV

| | |
|---|---|
| Fréquence de vibration de la buse (4) en hertz | 800 |
| Surpression de l'azote (4) en bars | 0,075 |
| Débit du produit à la sortie de la buse (4) en kg/h | 7,00 |
| Température de l'air à l'entrée de la tour (3) en °C | - 10°C |
| Température de l'air à la sortie de la tour (6) en °C | - 6°C |
| Température de l'air du lit fluide (9) en °C | - 10°C |

Après 10 minutes de fonctionnement, on obtient 840 g de sphérules de coumarine ayant un diamètre moyen ($d_{50}$) de 910 µm.

2 - Le produit obtenu présente la morphologie illustrée par la figure 4 (G = 50) et les caractéristiques suivantes :

- densité apparente non tassée = 0,50
- résistance à la compression (fc) = 1,25 kN/$m^2$ sous une pression de 4,8 kN/$m^2$
- vitesse de dissolution dans l'éthanol = 120 s.

Exemple 5

1 - On reproduit l'exemple 4 à la seule différence que la buse est statique.

Au bout de 8 minutes de fonctionnement, on obtient 520 g de sphérules de coumarine ayant un diamètre moyen ($d_{50}$) de 850 µm.

2 - Le produit obtenu présente la morphologie illustrée par la figure 5 (G = 100). On note une légère différence au niveau de la répartition granulométrique qui semble un peu moins uniforme.

Les caractéristiques physico-chimiques desdites sphérules sont les suivantes :

- densité apparente non tassée = 0, 47

Exemple 6

1 - La préparation des sphérules de coumarine est effectuée comme dans l'exemple 1 : les modifications des paramètres de procédé étant précisées dans le tableau suivant :

Tableau V

| | |
|---|---|
| Fréquence de vibration de la buse (4) en hertz | 1990 |
| Surpression de l'azote (4) en bars | 0,080 |
| Débit du produit à la sortie de la buse (4) en kg/h | 4,00 |
| Température de l'air à l'entrée de la tour (3) en °C | 10 |
| Température de l'air à la sortie de la tour (6) en °C | 12 |
| Température de l'air du lit fluide (9) en °C | 6 |

2 - Le produit obtenu présente les caractéristiques suivantes :

- densité apparente non tassée = 0,49
- vitesse de dissolution dans l'éthanol = 160 s.

Exemple 7

1 - 1 - On reproduit l'exemple 6 à la seule différence que la buse est statique.

2 - Le produit obtenu présente les caractéristiques suivantes :

- densité apparente non tassée = 0,45
- vitesse de dissolution dans l'éthanol = 135 s.

Exemple 8

1 - La préparation des sphérules de coumarine est effectuée comme dans l'exemple 1 : les modifications des paramètres de procédé étant précisées dans le tableau suivant :

Tableau VI

| | |
|---|---|
| Fréquence de vibration de la buse (4) en hertz | 1990 |
| Surpression de l'azote (4) en bars | 0,075 |
| Débit du produit à la sortie de la buse (4) en kg/h | 3,42 |
| Température de l'air à l'entrée de la tour (3) en °C | 12 |
| Température de l'air à la sortie de la tour (6) en °C | 14 |
| Température de l'air du lit fluide (9) en °C | 15 |

2 - Le produit obtenu présente les caractéristiques suivantes :

- densité apparente non tassée = 0,42
- vitesse de dissolution dans l'éthanol = 142 s.

## Revendications

**1.** Sphérules de la coumarine et/ou de ses dérivés.

**2.** Sphérules selon la revendication 1 caractérisées par le fait que la coumarine et/ou dérivé présente un point de fusion compris entre 50°C et 200°C, de préférence compris entre 50°C et 100°C.

**3.** Sphérules selon la revendication 1 et 2 caractérisées par le fait que la coumarine et/ou dérivé répond à la formule (I) suivante :

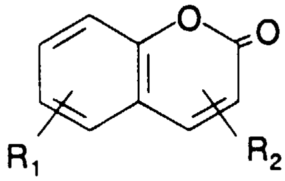

(I)

dans ladite formule (I), $R_1$, $R_2$, identiques ou différents représentent :

- un atome d'hydrogène,
- un radical alkyle linéaire ou ramifié ayant de 1 à 4 atomes de carbone,
- un radical alkoxy linéaire ou ramifié ayant de 1 à 4 atomes de carbone.

**4.** Sphérules selon l'une des revendications 1 à 3 caractérisées par le fait que la coumarine et/ou dérivé répond à la formule (I) dans laquelle $R_1$, $R_2$, identiques ou différents représentent un atome d'hydrogène, un radical méthyle ou méthoxy et, encore plus préférentiellement $R_1$ et $R_2$ représentent un atome d'hydrogène.

**5.** Sphérules selon l'une des revendications 1 à 4 caractérisées par le fait qu'elles ont une taille s'échelonnant entre 100 $\mu$m et 2000 $\mu$m mais se situant, de préférence, entre 300 $\mu$m et 1000 $\mu$m.

**6.** Sphérules selon l'une des revendications 1 à 5 caractérisées par le fait qu'elles ont une taille exprimée par le diamètre moyen ($d_{50}$) variant de 300 $\mu$m à 2 000 $\mu$m, de préférence entre 500 $\mu$m et 1 500 $\mu$m et encore plus préférentiellement entre 700 $\mu$m et 1 200 $\mu$m.

**7.** Sphérules selon l'une des revendications 1 à 6 caractérisées par le fait qu'elles ont une densité apparente (non tassée) variant entre 0,25 et 0,8, de préférence, entre 0,4 et 0,7.

**8.** Sphérules selon l'une des revendications 1 à 7 caractérisées par le fait qu'elles présentent une résistance à la compression comprise entre 500 et 10 000 $N/m^2$ , de préférence comprise entre 1 000 et 5 000 $N/m^2$, sous une contrainte inférieure à 10 000 $N/m^2$.

**9.** Sphérules selon l'une des revendications 1 à 8 caractérisées par le fait qu'elles ont une vitesse de dissolution dans l'éthanol inférieure à celle de la poudre cristallisée correspondante.

**10.** Sphérules selon la revendication 9 caractérisées par le fait que la vitesse de dissolution dans l'éthanol des sphérules de coumarine est inférieure à 170 secondes, de préférence, comprise entre 100 et 170 secondes, et encore plus préférentiellement comprise entre 100 et 130 secondes.

**11.** Procédé de préparation de sphérules de coumarine et/ou dérivés décrites dans l'une des revendications 1 à 10 caractérisé par le fait qu'il consiste à faire fondre si nécessaire la coumarine et/ou ses dérivés, puis à fragmenter la masse fondue en gouttelettes et à solidifier les gouttelettes obtenues dans un courant gazeux de refroidissement de telle sorte que les gouttelettes obtenues se solidifient en sphérules qui sont ensuite récupérées.

**12.** Procédé selon la revendication 11 caractérisé par le fait qu'il consiste à faire fondre si nécessaire la coumarine et/ou ses dérivés puis à faire passer la masse fondue dans une buse de façon à former des gouttelettes, à solidifier ces dernières en les laissant tomber dans une tour à contre-courant d'un gaz froid puis à récupérer les sphérules obtenues.

**13.** Procédé selon l'une des revendications 11 et 12 caractérisé par le fait que l'on fait fondre la coumarine et/ou dérivé à sa température de fusion, de préférence à une température supérieure à au plus 5°C par rapport à son point de fusion.

**14.** Procédé selon l'une des revendications 11 à 13 caractérisé par le fait que l'on transforme la masse fondue, en gouttelettes par passage au travers d'une buse.

**15.** Procédé selon la revendication 14 caractérisé par le fait que la buse utilisée est une buse à un seul trou ou une buse multi-trous avec un nombre de trous variant entre 1 et 100 trous.

**16.** Procédé selon la revendication 15 caractérisé par le fait que la buse utilisée comporte des perforations dont le diamètre varie entre 100 à 1 000 $\mu$m et se situe, de préférence, entre 200 et 600 $\mu$m.

**17.** Procédé selon l'une des revendications 15 et 16 caractérisé par le fait que la buse utilisée est une buse statique mais de préférence une buse soumise à un système de vibration électrique de haute fréquence, de préférence, de 500 à 10000 hertz.

**18.** Procédé selon l'une des revendications 11 à 17 caractérisé par le fait que les gouttelettes sont mises en contact avec un gaz froid, de préférence l'air, et plus particulièrement de l'air appauvri en oxygène, dont la température est choisie entre -30°C et 20°C, de préférence, entre -20°C et 10°C.

**19.** Procédé selon l'une des revendications 11 à 18 caractérisé par le fait que le temps de séjour de la gouttelette à la sortie de la buse et son arrivée dans le système de récupération se situe avantageusement, entre 1 et 10 secondes et plus préférentiellement entre 1 et 3 secondes.

**20.** Procédé selon l'une des revendications 11 à 19 caractérisé par le fait que l'on récupère les sphérules par tout moyen connu, de préférence selon la technique de lit fluide.

**Claims**

**1.** Spherules of coumarin and/or its derivatives.

**2.** Spherules according to claim 1, characterised in that the coumarin and/or its derivative has a melting point of between 50°C and 200°C, preferably between 50°C and 100°C.

**3.** Spherules according to claim 1 or claim 2, characterised in that the coumarin and/or its derivative has formula (I):

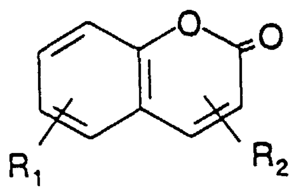
(I)

in which R1, R2, which may be identical or different, represent:

- a hydrogen atom;
- a linear or branched alkyl radical containing 1 to 4 carbon atoms;
- a linear or branched alkoxy radical containing 1 to 4 carbon atoms.

**4.** Spherules according to any one of claims 1 to 3, characterised in that the coumarin and/or its derivative has formula (I) in which R1, R2, which may be identical or different, represent a hydrogen atom or a methyl or methoxy radical, and preferably R1 and R2 represent a hydrogen atom.

**5.** Spherules according to any one of claims 1 to 4, characterised in that their particle size ranges from 100 µm to 2000 µm, and is preferably between 300 µm and 1000 µm.

**6.** Spherules according to any one of claims 1 to 5, characterised in that the particle size, expressed as the average diameter ($d_{50}$), varies between 300 µm and 2000 µm, preferably between 500 µm and 1500 µm, most preferably between 700 µm and 1200 µm.

**7.** Spherules according to any one of claims 1 to 6, characterised in that they have a bulk density (loose packed) of between 0.25 and 0.8, preferably between 0.4 and 0.7.

**8.** Spherules according to any one of claims 1 to 7, characterised in that they have a compressive strength of between 500 and 10000 $N/m^2$, preferably between 1000 and 5000 $N/m^2$, at a stress of less than 10000 $N/m^2$.

**9.** Spherules according to any one of claims 1 to 8, characterised in that they have a dissolution rate in ethanol which is lower than that of a corresponding crystalline powder.

**10.** Spherules according to claim 9, characterised in that the dissolution rate in ethanol of spherules of coumarin is

less than 170 seconds, preferably between 100 and 170 seconds, and most preferably between 100 and 130 seconds.

**11.** A process for the preparation of spherules of coumarin and/or its derivatives as described in any one of claims 1 to 10, characterised in that the coumarin and/or its derivative is melted if necessary then the molten mass is fragmented into droplets and the droplets obtained are solidified in a gaseous cooling current to solidify them into spherules which are then recovered.

**12.** A process according to claim 11, characterised in that it consists of melting the coumarin and/or its derivative if necessary then passing the molten mass through a nozzle to form droplets, solidifying the latter by allowing them to fall in a tower against a counter-current of a cold gas, then recovering the spherules obtained.

**13.** A process according to any one of claims 1 to 12, characterised in that the coumarin and/or its derivative is melted at its melting point, preferably at a temperature which is at most 5°C higher than its melting point.

**14.** A process according to any one of claims 1 to 13, characterised in that the molten mass is transformed into droplets by passage through a nozzle.

**15.** A process according to claim 14, characterised in that the nozzle is a single-holed or a multi-holed nozzle in which the number of holes is between 1 and 100.

**16.** A process according to claim 15, characterised in that the nozzle contains perforations with a diameter of between 100 and 1000 µm, preferably between 200 and 600 µm.

**17.** A process according to claim 15 or claim 16, characterised in that the nozzle is a static nozzle, preferably a nozzle which is electrically vibrated at high frequency, preferably 500 to 10000 hertz.

**18.** A process according to any one of claims 11 to 17, characterised in that the droplets are brought into contact with a cold gas, preferably air, more particularly oxygen-depleted air, in which the temperature is between -30°C and 20°C, preferably between -20°C and 10°C.

**19.** A process according to any one of claims 11 to 18, characterised in that the residence time of the droplet between the nozzle outlet and its arrival at the recovery system is advantageously between 1 and 10 seconds, more preferably between 1 and 3 seconds.

**20.** A process according to any one of claims 11 to 19, characterised in that the spherules are recovered using any known means, preferably using a fluidised bed technique.

## Patentansprüche

**1.** Kügelchen aus Cumarin und/oder aus seinen Derivaten.

**2.** Kügelchen nach Anspruch 1, dadurch gekennzeichnet, daß das Cumarin und/oder deren Derivate einen Schmelzpunkt zwischen 50°C und 200°C, vorzugsweise zwischen 50 °C und 100 °C, haben.

**3.** Kügelchen nach Anspruch 1 oder 2, dadurch gekennzeichnet, daß das Cumarin und/oder deren Derivate der folgenden Formel (I) entsprechen:

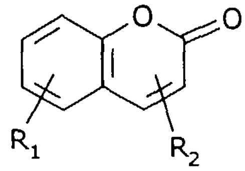

(I)

wobei in der genannten Formel (I) die gleichen oder verschiedenen $R_1$ und $R_2$ bedeuten:

- ein Wasserstoffatom,
- einen linearen oder verzweigten Alkylrest mit 1 bis 4 Kohlenstoffatomen,
- einen linearen oder verzweigten Alkoxyrest mit 1 bis 4 Kohlenstoffatomen.

**4.** Kügelchen nach einem der Ansprüche 1 bis 3, dadurch gekennzeichnet, daß das Cumarin und/oder deren Derivate der Formel (I) entsprechen, in der die gleichen oder verschiedenen $R_1$ und $R_2$ ein Wasserstoffatom oder einen Methyl- oder Methoxyrest bedeuten, wobei $R_1$ und $R_2$ noch spezieller ein Wasserstoffatom bedeuten.

**5.** Kügelchen nach einem der Ansprüche 1 bis 4, dadurch gekennzeichnet, daß sie eine Größe haben, die zwischen 100 µm und 2.000 µm, aber vorzugsweise zwischen 300 µm und 1.000 µm, liegt.

**6.** Kügelchen nach einem der Ansprüche 1 bis 5, dadurch gekennzeichnet, daß sie eine durch den mittleren Durchmesser ($d_{50}$) definierte Größe haben, die von 300 µm bis 2.000 µm reicht, vorzugsweise zwischen 500 µm und 1.500 µm und noch spezieller zwischen 700 µm und 1.200 µm liegt.

**7.** Kügelchen nach einem der Ansprüche 1 bis 6, dadurch gekennzeichnet, daß sie eine (unverdichtete) Schüttdichte zwischen 0,25 und 0,8, vorzugsweise zwischen 0,4 und 0,7, haben.

**8.** Kügelchen nach einem der Ansprüche 1 bis 7, dadurch gekennzeichnet, daß sie eine Druckfestigkeit zwischen 500 und 10.000 $N/m^2$, vorzugsweise zwischen 1.000 und 5.000 $N/m^2$, bei einer Belastung unter 10.000 $N/m^2$ haben.

**9.** Kügelchen nach einem der Ansprüche 1 bis 8, dadurch gekennzeichnet, daß sie eine Lösungsgeschwindigkeit in Ethanol haben, die kleiner als die des entsprechenden kristallisierten Pulvers ist.

**10.** Kügelchen nach Anspruch 9, dadurch gekennzeichnet, daß die Lösungsgeschwindigkeit der Cumarinkügelchen in Ethanol unter 170 Sekunden, vorzugsweise zwischen 100 und 170 Sekunden und noch spezieller zwischen 100 und 130 Sekunden, liegt.

**11.** Verfahren zur Herstellung von Kügelchen aus Cumarin und/oder deren Derivate nach einem der Ansprüche 1 bis 10, dadurch gekennzeichnet, daß es darin besteht, das Cumarin und/oder deren Derivate falls erforderlich zu schmelzen, dann die Schmelzmasse in Tröpfchen zu zerteilen und die erhaltenen Tröpfchen in einem gasförmigen Kühlstrom derart erstarren zu lassen, daß die erhaltenen Tröpfchen zu Kügelchen erstarren, die anschließend aufgefangen werden.

**12.** Verfahren nach Anspruch 11, dadurch gekennzeichnet, daß es darin besteht, das Cumarin und/oder deren Derivate falls erforderlich zu schmelzen, dann die Schmelzmasse durch eine Düse zu leiten, so daß Tröpfchen gebildet werden, letztere erstarren zu lassen, indem sie in einen Turm mit Gegenstrom eines kalten Gases fallengelassen werden, und dann die erhaltenen Kügelchen aufzufangen.

**13.** Verfahren nach Anspruch 11 oder 12, dadurch gekennzeichnet, daß man das Cumarin und/oder deren Derivate bei seiner Schmelztemperatur, vorzugsweise bei einer Temperatur, die höchstens 5°C über seinem Schmelzpunkt liegt, schmelzen läßt.

**14.** Verfahren nach einem der Ansprüche 11 bis 13, dadurch gekennzeichnet, daß man die Schmelzmasse zu Tröpfchen formt, indem man sie durch eine Düse leitet.

**15.** Verfahren nach Anspruch 14, dadurch gekennzeichnet, daß die verwendete Düse eine Düse mit einer einzigen Öffnung oder eine Düse mit mehreren Öffnungen ist, wobei die Zahl der Öffnungen von 1 bis 100 Öffnungen reicht.

**16.** Verfahren nach Anspruch 15, dadurch gekennzeichnet, daß die verwendete Düse Perforierungen enthält, deren Durchmesser zwischen 100 und 1.000 µm und vorzugsweise zwischen 200 und 600 µm liegt.

**17.** Verfahren nach Anspruch 15 oder 16, dadurch gekennzeichnet, daß die verwendete Düse eine statische Düse ist, vorzugsweise aber eine Düse, die einem System elektrischer Vibrationen hoher Frequenz, vorzugsweise von 500 bis 10.000 Hertz, ausgesetzt ist.

**18.** Verfahren nach einem der Ansprüche 11 bis 17, dadurch gekennzeichnet, daß die Tröpfchen mit einem kalten

Gas, vorzugsweise Luft und noch spezieller sauerstoffarmer Luft, zusammengebracht werden, dessen Temperatur zwischen -30°C und 20°C, vorzugsweise zwischen -20°C und 10°C, gewählt ist.

**19.** Verfahren nach einem der Ansprüche 11 bis 18, dadurch gekennzeichnet, daß die Verweilzeit des Tröpfchens zwischen dem Düsenaustritt und seiner Ankunft in der Auffangvorrichtung vorteilhafterweise zwischen 1 und 10 Sekunden und noch spezieller zwischen 1 und 3 Sekunden liegt.

**20.** Verfahren nach einem der Ansprüche 11 bis 19, dadurch gekennzeichnet, daß man die Kügelchen mit allen bekannten Mitteln, vorzugsweise mit der Fließbetttechnik, auffängt.

Figure 1

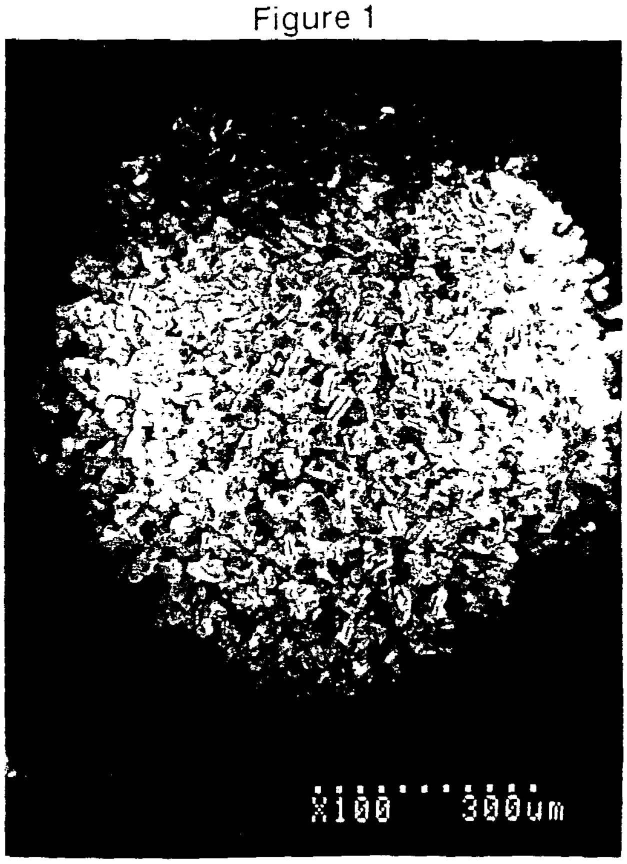


Figure 2

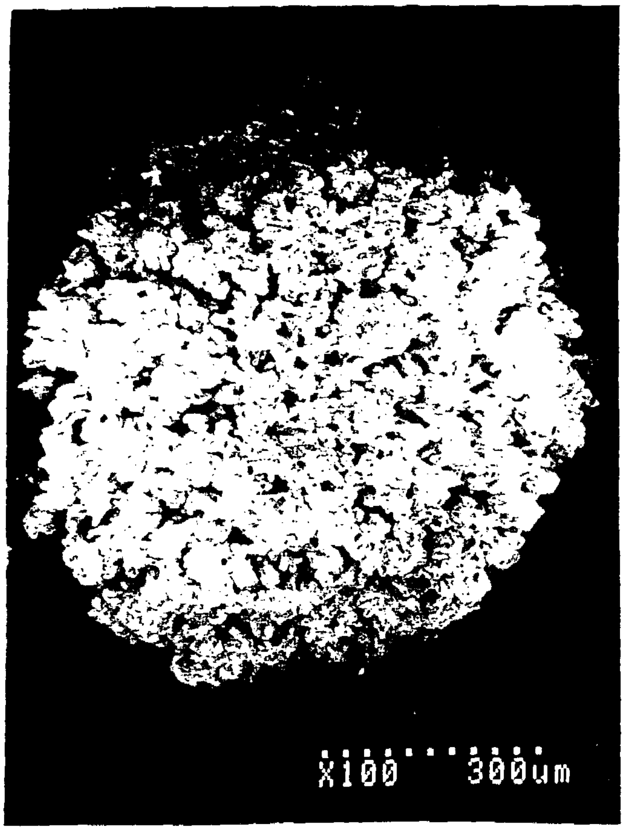

Figure 3

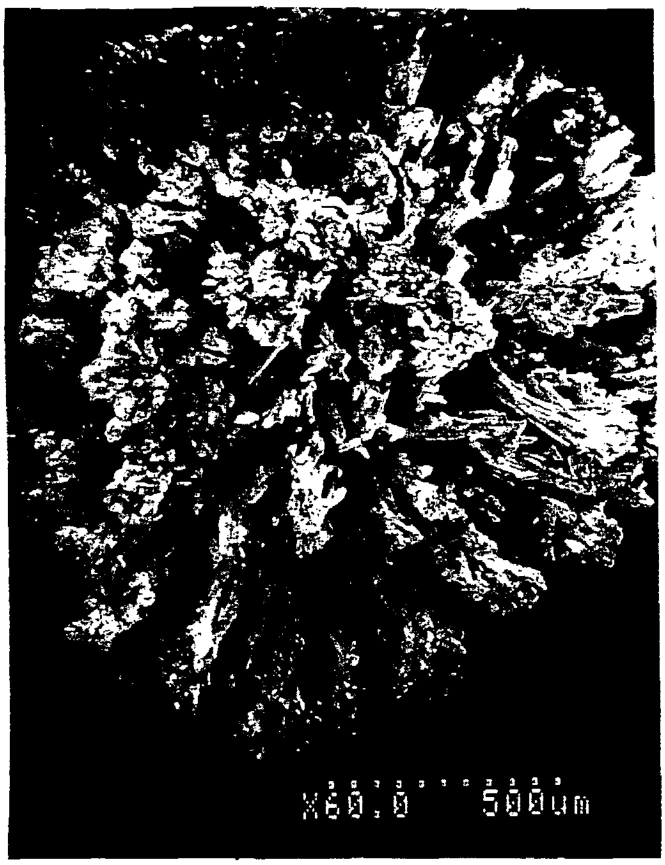

Figure 4

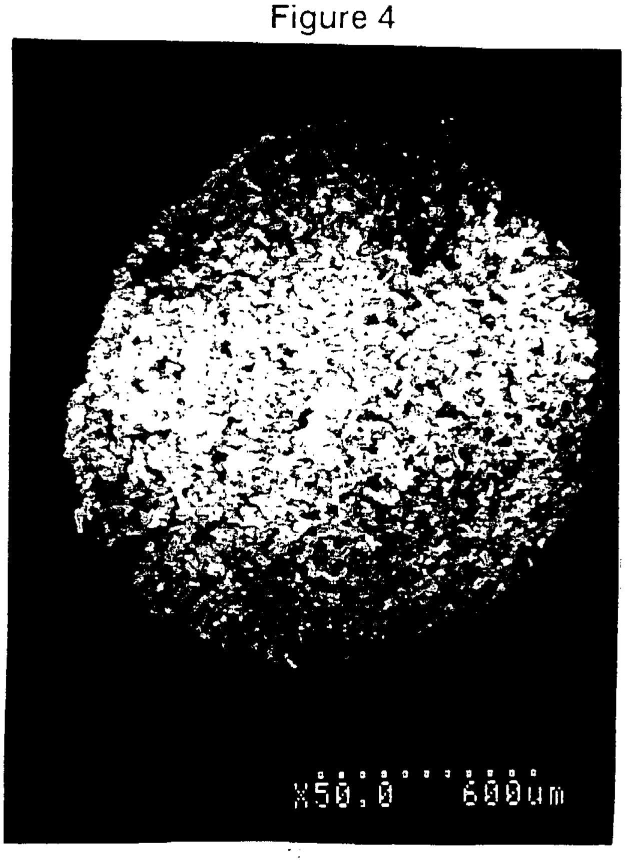


Figure 5

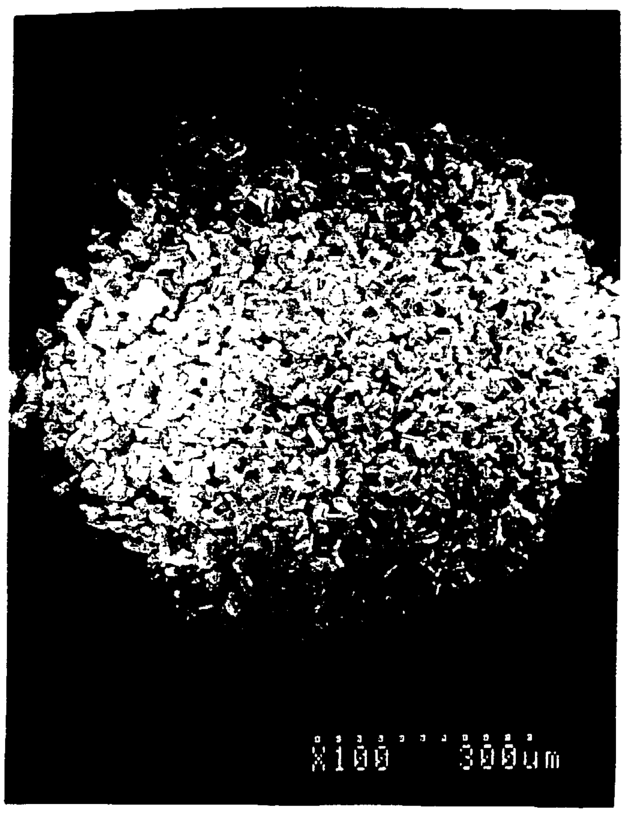

Figure 6

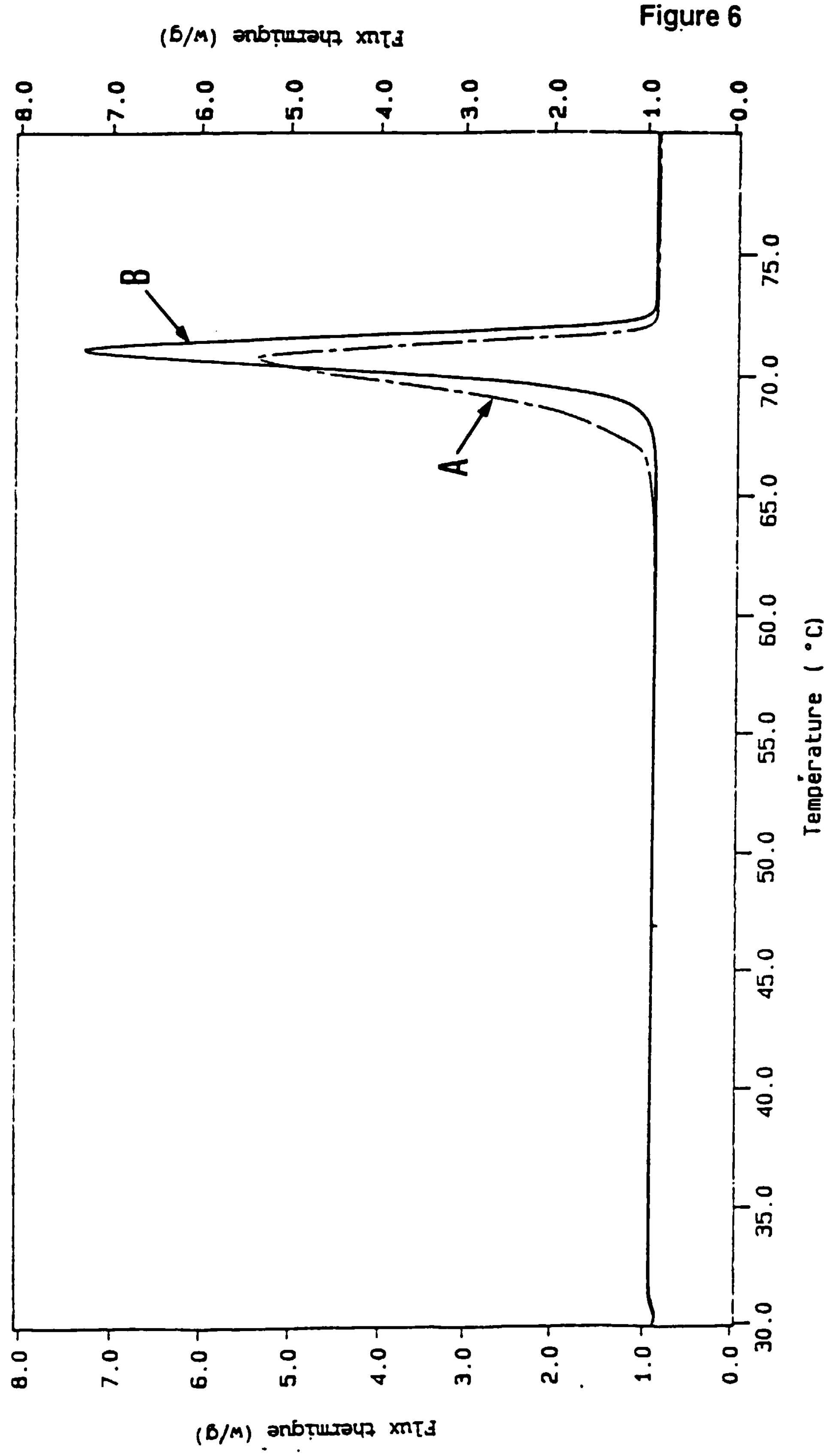
Flux thermique (w/g)
8.0
7.0
6.0
5.0
4.0
3.0
2.0
1.0
0.0
30.0
35.0
40.0
45.0
50.0
55.0
60.0
65.0
70.0
75.0
Température ( °C)
A
B

Figure 7

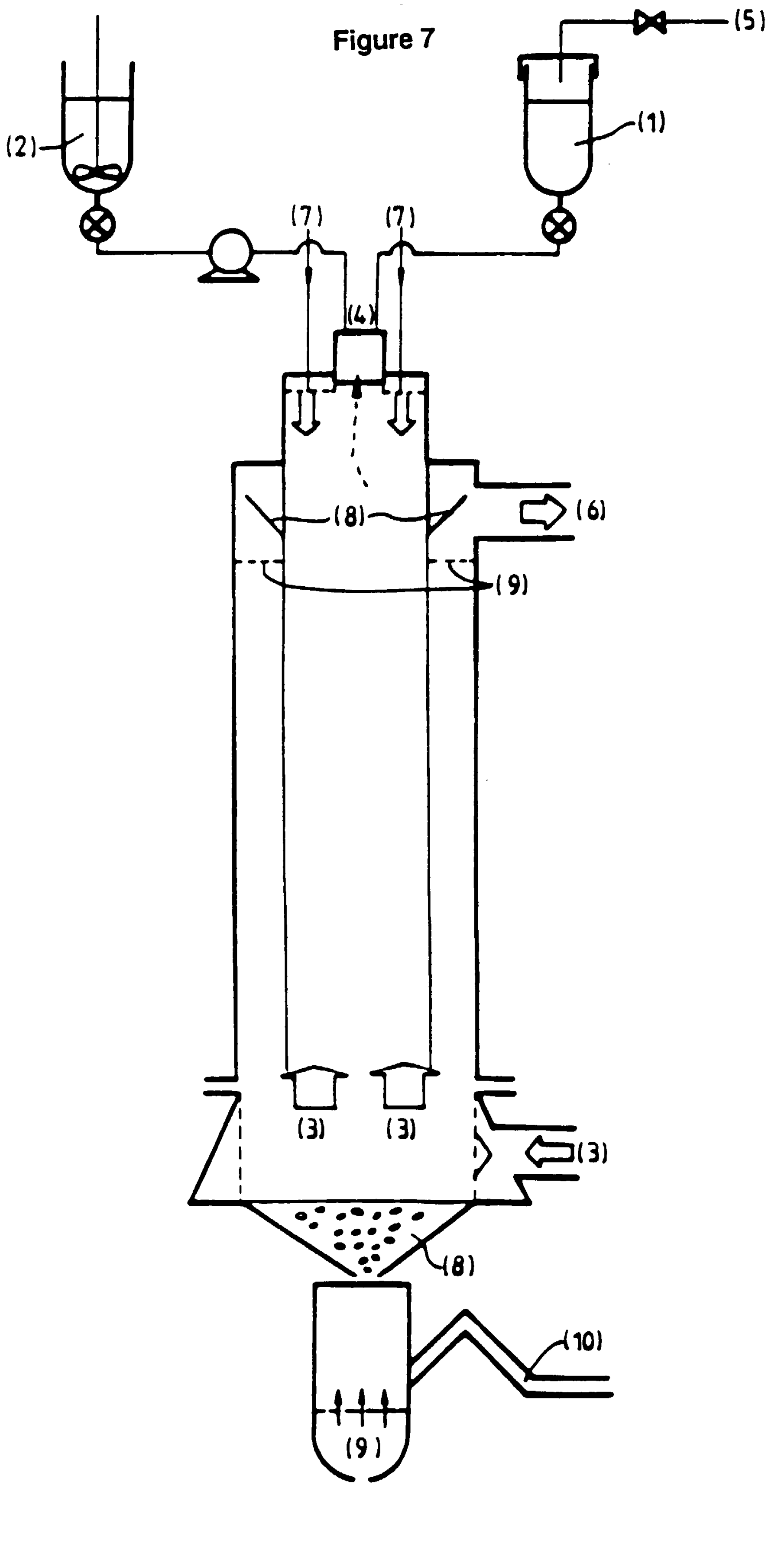

Figure 8